# EUROPÄISCHE PATENTANMELDUNG

(11) **EP 4 268 744 A1**
(43) Veröffentlichungstag der Anmeldung: **01.11.2023**
(21) Anmeldenummer: 22170201.2
(22) Anmeldetag: 27.04.2022
(51) Int. Cl.: A61B 18/02

(54) **MEDIZINISCHES KRYOTHERAPIE-GERÄT MIT WÄRMEÜBERTRAGUNGSKÖRPER**

(71) Anmelder: Cryovasc GmbH, 10119 Berlin (DE)
(72) Erfinder: HEINER, Wilfried, 10119 Berlin (DE); KERN, Karoline, 10119 Berlin (DE)
(74) Vertreter: Stütz, Jan

(57) **Zusammenfassung**

Die vorliegende Erfindung betrifft ein medizinisches Gerät (1) zur Kryotherapie umfassend einen Katheter (3) zur Kälteablation. Der Katheter (3) weist einen wenigstens teilweise flexiblen, rohrförmigen Schaft (5) zum Einführen des Katheters (3) in ein Körpergefäß auf, wobei der Schaft (5) ein erstes Lumen (23) zum Transportieren eines Kühlmediums zu einem distalen Ende (11) des Schafts (5) und ein zweites Lumen (25) zum Abtransportieren des Kühlmediums vom distalen Ende (11) des Schafts (5) umfasst. Das erste Lumen (23) ist an einem proximalen Ende des Schafts (5) mit einer Kühlmediumquelle (21) verbunden. Der Katheter (3) weist weiterhin eine expandierbare Kühlkammer (29) auf. Diese Kühlkammer (29) ist am distalen Ende (11) des Schafts (5) angeordnet und umschließt einen Endabschnitt des distalen Endes (11) des Schafts (5) und/oder einen einem Endabschnitt des distalen Endes (11) des Schafts (5) zugeordneten ersten Lumenabschnitt des ersten Lumens (23). Der Kühlkammer (29) ist das Kühlmedium über das erste Lumen (23) mittels wenigstens einer Düse (33) zuführbar. Aus der Kühlkammer (29) ist das Kühlmedium über das zweite Lumen (25) abtransportierbar. Thermische Energie ist über eine Außenwand (29b) der expandierten Kühlkammer (29) übertragbar, so dass eine Kryotherapie an einem Behandlungsort durchführbar ist. Erfindungsgemäß umfasst der Endabschnitts am distalen Ende (11) des Schafts (5) einen Wärmeübertragungskörper (27), welcher ein inneres Element (39) und ein das innere Element (39), insbesondere hut-, kappen- oder mantelförmig, umschließendes äußeres Element (41) aufweist, wobei durch das Zusammenwirken des inneren Elements (39) und des äußeren Elements (41) der erste Lumenabschnitt des ersten Lumens (23) gebildet ist. Zudem nützt die wenigstens eine Düse (33) den Joule-Thomson-Effekt. Wenigstens ein Teil des Endabschnitts am distalen Ende (11) des Schafts (5) ist mit dem Inneren (29a) der Kühlkammer (29) in wärmeleitendem Kontakt. Dies geschieht zur Kühlung des im ersten Lumen (23) geführten Kühlmediums durch das dem Inneren (29a) der Kühlkammer (29) zugeführte Kühlmedium.

## Beschreibung

Die vorliegende Erfindung betrifft ein Kryotherapie-Gerät insbesondere zur intravaskulären Kryotherapie, zum Beispiel zur Ablation von Körpergewebe, zur Denervierung perivaskulärer Nerven oder zur Stabilisierung von Atherosklerotischen Plaques.

Beispielsweise wird zur Behandlung von Bluthochdruck und Hypertonie mit einem Kryotherapie-Gerät durch eine Denervierung der renalen perivaskulären Nerven die den Bluthochdruck wenigstens mitverursachende Aktivität dieser Nerven unterdrückt oder ausgeschaltet.

Ein solches medizinisches Gerät und ein Verfahren zum Betreiben dieses Geräts ist in der europäischen Patentanmeldung EP 3 708 100 A1 offenbart.

Dieses bekannte Gerät zeigt und beschreibt ein medizinisches Gerät zur Denervierung renaler perivaskulärer Nerven und weist einen Katheter mit einem Schaft und einem in diesem Schaft verlaufenden inneren Lumen zum Zuführen eines Kühlmediums auf. Am distalen Ende des Schafts ist ein Kryoballon zur Kälteablation der Nerven angeordnet. Im Anwendungsfall, also während einer Behandlung, erfolgt eine Berührung der renalen Arterie mit dem Kryoballon, wodurch die beabsichtigten Kryoablation der Nerven durchgeführt wird.

Das bekannte Gerät weist jedoch den Nachteil auf, dass die zur Ablationsbehandlung erforderliche tiefe Temperatur bzw. die erforderliche Kühlleistung nicht immer und insbesondere nicht über die gewünschte Länge des Therapie-Bereichs erreicht werden kann.

Dieser Nachteil tritt auch bei anderen Kryotherapie-Geräten aus dem Stand der Technik auf, insbesondere bei Geräten, deren Einsatzgebiet kleine Blutgefäße betrifft.

Aus dem Stand der Technik sind Geräte bekannt, welche ebenfalls dieses Problem adressieren, wie beispielsweise EP 1 129 669 A1. Das in diesem Stand der Technik beschriebene Gerät ist ein Kryoablationskatheter, welcher ein röhrenförmiges Element zum Zuführen eines Kühlmediums umfasst. Dieses Element ist dabei um den äußeren Umfang des distalen Endes des Katheters gewunden, welcher wiederum von einem Ballon umschlossen wird. Durch das Austreten des Kühlmittels aus dem röhrenförmigen Element wird dem Inneren des Ballons Wärme entzogen, sodass der Ballon zur Kryotherapie eingesetzt werden kann. Gerade für kleinere Blutgefäße ist die Herstellung eines entsprechend dimensionierten Katheters nach diesem Stand der Technik schwer möglich und auch dessen Kühlleistung wäre in diesem Falle verbesserungsbedürftig.

Der vorliegenden Erfindung liegt daher die Aufgabe zugrunde, ein medizinisches Gerät zur Kryotherapie insbesondere intravaskulären Kryotherapie zur Ablation von Körpergewebe, Denervierung, oder Stabilisierung von Atherosklerotischen-Plaques bereit zu stellen, welches eine verbesserte Kühlleistung besitzt und kostengünstiger herzustellen ist.

Die voranstehende Aufgabe wird mit einem medizinischen Gerät gemäß Patentanspruchs 1 und ein Verfahren gemäß Anspruch 12 gelöst.

Ein medizinisches Gerät zur Kryotherapie insbesondere zur intravaskulären Kryotherapie - zur Ablation von Körpergewebe, Denervierung oder Stabilisierung von Atherosklerotischen-Plaques umfasst einen Katheter zur Kälteablation. Der Katheter weist einen wenigstens teilweise flexiblen, rohrförmigen Schaft zum Einführen des Katheters in ein Körpergefäß auf, wobei der Schaft ein erstes Lumen zum Transportieren eines Kühlmediums zu einem distalen Ende des Schafts und ein zweites Lumen zum Abtransportieren des Kühlmediums vom distalen Ende des Schafts umfasst. Das Körpergefäß ist beispielsweise eine renale Arterie eines Patienten.

Das erste Lumen ist an einem proximalen Ende des Schafts mit einer Kühlmediumquelle verbunden. Vorzugsweise ist das erste Lumen ein inneres Lumen und das zweite Lumen ein äußeres Lumen, wobei insbesondere das äußere Lumen das innere Lumen, beispielsweise in einer konzentrischen Anordnung, umgibt. Der Katheter weist weiterhin eine expandierbare Kühlkammer, insbesondere einen Kryoballon, auf.

Diese Kühlkammer ist am distalen Ende des Schafts angeordnet und umschließt einen Endabschnitt des distalen Endes des Schafts und/oder einen ersten Lumenabschnitt des ersten Lumens, welcher einem Endabschnitt des distalen Endes des Schafts zugeordnet ist und welcher insbesondere ein distaler Lumenabschnitt des ersten Lumens ist. Der Kühlkammer ist das Kühlmedium über das erste Lumen mittels wenigstens einer Düse bzw. Drossel zuführbar. Die Kühlkammer ist insbesondere unter Zufuhr des Kühlmediums expandierbar, wobei thermische Energie über eine Außenwand der Kühlkammer übertragbar ist, so dass eine Kryotherapie an einem Behandlungsort durchführbar ist. Aus der Kühlkammer ist schließlich das Kühlmedium über das zweite Lumen abtransportierbar, wobei dieser Abtransport vorzugsweise nach der Durchführung der Kryotherapie am Behandlungsort, also beispielsweise im Bereich der renalen perivaskulären Nerven, ausgeübt wird. Diese Kälteablations-Behandlung wird durch Übertragung von Wärmeenergie vom Behandlungsort an das Kühlmedium über die Außenwand der Kühlkammer bedingt.

Gemäß der vorliegenden Erfindung nutzt die wenigstens eine Düse den Joule-Thomson-Effekt. Außerdem ist wenigstens ein Teil des Endabschnitts am distalen Ende des Schafts mit dem Inneren der Kühlkammer in wärmeleitendem Kontakt zur Kühlung des im ersten Lumen geführten Kühlmediums durch das dem Inneren der Kühlkammer zugeführte Kühlmedium verbunden.

Der Teil des Endabschnitts am distalen Ende des Schafts kann dabei wenigstens ein überwiegender Teil des ersten Lumenabschnitts oder aber der gesamte erste Lumenabschnitt des ersten Lumens sein, der von der expandierbaren Kühlkammer umschlossen ist.

Der Endabschnitt am distalen Ende des Schafts umfasst erfindungsgemäß einen Wärmeübertragungskörper oder bildet einen Wärmeübertragungskörper.

Dabei weist dieser Wärmeübertragungskörper ein inneres Element und ein das innere Element umschließendes äußeres Element auf. Das äußere Element ist erfindungsgemäß hut-,kappen- oder mantelförmig ausgebildet. Dadurch ist durch das Zusammenwirken des inneren Elements und des äußeren Elements der erste Lumenabschnitt des ersten Lumens gebildet.

Vorzugsweise ist das äußere Element und/ oder das innere Element wenigstens teilweise aus einem Material mit großer Wärmeleitfähigkeit hergestellt. Zusätzlich, oder auch alternativ, kann das innere Element eine Kernstruktur umfassen oder eine Kernstruktur sein. Ein solcher zweiteiliger Aufbau des Wärmeübertragungskörpers aus einem inneren und einem äußeren Element führt zu einer einfachen Herstellweise. Vorzugsweise ist das innere Element im äußeren Element spielfrei eingepasst oder einführbar.

Dadurch, dass die wenigstens eine Düse eine den Joule-Thomson-Effekt nutzende Düse ist und wenigstens ein Teil des Endabschnitts am distalen Ende des Schafts mit dem Inneren der Kühlkammer in wärmeleitendem Kontakt zur Kühlung des im ersten Lumen geführten Kühlmedium durch das dem Inneren der Kühlkammer zugeführte Kühlmedium ist, ist ein medizinisches Gerät geschaffen, mit dem mit einer erhöhten Kühlleistung eine noch effizientere Behandlung bei der Denervierung insbesondere renaler perivaskulärer Nerven durchführbar ist.

Vorzugsweise ist das von der Kühlmediumquelle dem ersten Lumen zugeführte Kühlmedium gasförmig und wird verflüssigt durch die Kühlung des Kühlmediums im ersten Lumen - infolge des Übergangs von thermischer Energie zwischen dem ersten Lumenabschnitt des ersten Lumens und dem Inneren der Kühlkammer. Das Kühlmedium ist bevorzugt bis zum Eintreten in den Kühlkammerbereich gasförmig, und wird mit Hilfe der Kühlung durch den Wärmeübertragungskörper in den flüssigen Aggregatszustand überführt bzw. ist im Anwendungsfall überführbar. Die Kühlung bzw. der Übergang der thermischen Energie findet insbesondere im ersten Lumenabschnitt des ersten Lumens statt.

Als Kühlmedium hat sich Distickstoffmonoxid (N₂O) als besonders geeignet herausgestellt, wobei aber auch andere Kühlmedien zum Einsatz kommen können. Prinzipiell ist jedes Gas, das expandierbar und verflüssigbar ist, als Kühlmedium verwendbar. Der Eintrag des Kühlmediums in das Innere der Kühlkammer mittels der Düse ist dann insbesondere von einer erneuten Aggregatszustandsänderung begleitet, nämlich von flüssig nach gasförmig, wobei bei dieser Aggregatszustandsänderung Verdampfungsenergie benötigt wird, die als Wärmemenge der unmittelbaren Umgebung, also im Wesentlichen dem Inneren der Kühlkammer, entzogen wird. Auf diese Weise kommt es zu einer weiteren Abkühlung des Inneren der Kühlkammer. Die Erzeugung der Kälte durch den Joule-Thomson-Effekt wird also verstärkt durch den genannten Entzug von Wärmeenergie infolge der Aggregatszustandsänderung des Kühlmediums von flüssig nach gasförmig.

Eine besonders vorteilhafte Ausgestaltung der Erfindung sieht vor, dass der erste Lumenabschnitt des ersten Lumens eine vergrößerte, vorzugsweise eine wesentlich vergrößerte, Grenz- oder Oberfläche zur Übertragung der thermischen Energie vom Innern der Kühlkammer an das erste Lumen im Vergleich zu einem zweiten Lumenabschnitt, der außerhalb der Kühlkammer angeordnet ist, aufweist, wobei dieser zweite Lumenabschnitt in axialer Richtung des ersten Lumens die gleiche Erstreckung in Längsrichtung wie der erste Lumenabschnitts hat. Durch diese vergrößerte Grenz- oder Oberfläche kann die axiale Fließgeschwindigkeit, insbesondere eine axiale Komponente der Kältemittel-Fließgeschwindigkeit, des Kältemittels im Bereich des ersten Lumenabschnitts des ersten Lumens gegenüber der axialen Fließgeschwindigkeit im zweiten Lumenabschnitt herabgesetzt sein. Die Übertragung der thermischen Energie von dem ersten Lumen bewirkt insbesondere eine Übertragung an bzw. eine Aufnahme durch das Kühlmedium, das sich zu diesem Zeitpunkt in der Kühlkammer befindet. Durch diese Erfindung ist insbesondere eine Wärmeübertragungsanordnung vorgeschlagen, die zu einer weiteren Steigerung der Kühleffizienz führen kann.

Vorzugsweise ist der erste Lumenabschnitt des ersten Lumens helixförmig um die Kernstruktur gewickelt. Dabei können die Kernstruktur und die helixförmige Anordnung des ersten Lumenabschnitts von einem Wärmeübertragungskörper umfasst oder eingeschlossen sein. Durch diese Anordnung des ersten Lumenabschnitts des ersten Lumens lässt sich gemäß einer Ausführung die zur Wärmeübertragung verwendete Grenz- oder Oberfläche im Inneren der Kühlkammer vergrößern, ohne dass notwendigerweise die Strömungsgeschwindigkeit, generell oder in einem wesentlichen Umfang, in irgendeinem Abschnitt des ersten Lumens geändert werden muss.

Eine besonders bevorzugte Ausgestaltung der Helixform sieht vor, dass das erste Lumen, insbesondere das Kühlmedium im ersten Lumen, im ersten Lumenabschnitt nach einem einfachen, vollständigen Umlauf um die Kernstruktur einen Streckenabschnitt in axialer Richtung durchläuft, der nicht größer ist, als der zweifache Durchmesser des ersten Lumens. Vorzugsweise ist dieser Streckenabschnitt geringfügig größer als der einfache Durchmesser des ersten Lumens. In einer Querschnittsansicht entspricht dieser Streckenabschnitt der Distanz der Zentren einer ersten Schnittfläche und einer zweiten Schnittfläche des ersten Lumens, wobei zwischen erster und zweiter Schnittfläche ein einfacher vollständiger Umlauf des ersten Lumens um die Kernstruktur liegt.

Durch diese besonders eng gewundene Helixanordnung stellt sich eine besondere Oberflächenvergrößerung ein, aus der in entsprechender Weise eine besonders wirkungsvolle Wärmeübertragungsfläche resultiert.

Eine vorteilhafte Ausgestaltung der Erfindung ist dadurch gekennzeichnet, dass der erste Lumenabschnitt des ersten Lumens als Vertiefung an der Oberfläche des inneren Elements dargestellt ist. Diese Oberfläche des inneren Elements kann als eine Zylindermantelfläche ausgebildet sein. Vorzugsweise ist diese Vertiefung als helixförmige oder spiralförmige Nut dargestellt. Außerdem kann die Vertiefung einen U-förmigen Querschnitt aufweisen. Ein solcher Aufbau ist für eine besonders einfache Herstellung des Wärmeübertragungskörpers, mit vorzugsweise besonders geringen Abmessungen, hilfreich. Ein derart verkleinerter Aufbau des Katheters ist günstig, um noch weitergehend eine schonende Behandlungsmethodik einzusetzen.

Gemäß einer weiteren Ausführungsform kann vorgesehen sein, dass der erste Lumenabschnitt des ersten Lumens durch eine in das erste Lumen am distalen Ende eingeschobene Kernstruktur mit besonders bevorzugt helixförmiger Vertiefung gebildet wird.

Der Wärmeübertragungskörper wird demnach von einem in das erste Lumen eingeschobenen Kern bzw. Kernelement zusammen mit der mantelförmigen Wand eines schlauchförmigen Elementes gebildet, welches das erste Lumen an sich definiert. Die bevorzugt in der Kernstruktur vorhandene wenigsten eine Nut zusammen mit der die Kernstruktur mantelförmig umschließende innere Oberfläche des schlauchförmigen Elementes definiert den ersten Lumenabschitt des ersten Lumens. Hierdurch strömt das zunächst gasförmige Kühlmedium und wird dabei von außen durch die Wand des schlauchförmigen Elementes gekühlt und bevorzugt verflüssigt, um am Ende des Kernelements über eine bevorzugt zwischen Kernelement und schlauchförmigem Element gebildeten Düse zu verdampfen und eine Kühlung der Kühlkammer zu bewirken.

Gemäß einer besonders bevorzugten Ausführung ist vorgesehen, dass die Tiefe der Nut in Richtung der Düse abnimmt. Hierdurch erhöht sich die Geschwindigkeit und der statische Druck wird verringert. Es kann aber auch ein konstanter oder sich Richtung Düse erweiternder Querschnitt der Nut vorgesehen sein. Diese Variationsmöglichkeiten gelten für alle Ausführungsvarianten der Erfindung.

In einer besonderen ebenfalls flexibel kombinierbaren Ausführungsform kann der erste Lumensabschnitt des ersten Lumens auch insgesamt als Düse z.B. als Venturi-Düse ausgeführt sein. In diesem Falle erfolgt im Übergangsbereich zwischen dem ersten Lumen und dem ersten Lumensabschnitt eine Querschnittsverengung bis hin zu einem minimalen Querschnitt, bei dem die Nut den geringsten Querschnitt aufweist. Daran anschließend erfolgt eine Erweiterung des Querschnittes der Nut bis hin zum Austritt aus der Düse in die Kühlkammer. Es kann vorgesehen sein, dass im Bereich des geringsten Querschnittes eine Öffnung zum Ansaugen von verdampftem Kühlmedium aus der Kühlkammer vorgesehen ist.

Es kann vorgesehen sein, dass in der Kühlkammer, insbesondere im Kryoballon, durch den Austritt des Kühlmediums aus der Düse ein gegenüber dem Fluiddruck im ersten Lumen niedrigerer Druck einstellbar ist. Dieser Druck ist in günstiger Weise kleiner als ein der Festigkeit der Außenwand der Kühlkammer zugeordneter oder zuordenbarer Maximaldruck. Auf diese Weise kann eine Überbelastung der Außenwand mit der Gefahr eines Bruchs derselben vermieden werden. Besonders vorteilhaft ist die Düse eine Venturidüse, was zu einer Steigerung des Abkühleffekts bei der dadurch erhöhten Expansion des Kühlmediums beim Eintritt in die Kühlkammer führen kann.

Gemäß einer Ausführungsform des medizinischen Geräts ist die Außenwand der Kühlkammer, insbesondere die Hülle des Kryoballons, aus einem dünnwandigen dehnbaren Material gebildet und doppelwandig ausgestaltet. Um die Sicherheit bei der Behandlung, insbesondere zur Vermeidung einer Embolie während der Behandlung infolge eines Defekts der Außenwand der Kühlkammer und eines Kühlmediumeintritts in die Arterie des Patienten, zu erhöhen, kann wenigstens ein Sensor zur Erfassung des Zustands dieser Außenwand umfasst sein. Dies ist beispielsweise durch einen Sensor realisierbar, der wenigstens einen Parameter im durch die innere und die äußere Wand definierten Zwischenraum der Außenwand überwacht. Vorteilhafterweise ist vorgesehen, dass bei einem Erkennen eines solchen Defekts durch den Sensor wenigstens ein Warnsignal durch eine Kontrolleinheit des medizinischen Geräts ausgegeben wird, und besonders bevorzugt wird automatisch die Kühlmediumquelle abgeschaltet, so dass kein weiteres Kühlmedium in die Kühlkammer eingetragen werden kann.

In alternativer Weise oder zusätzlich zur doppelwandigen Ausgestaltung kann die Außenwand der Kühlkammer, insbesondere die Hülle des Kryoballons, wenigstens im Betriebszustand des medizinischen Gerätes aus einem Material umfassend eine Formgedächtnislegierung gebildet und/oder von einem Korb wenigstens teilweise umgeben sein. Auch dieser Korb kann aus einem Material gebildet sein, das eine Formgedächtnislegierung, zum Beispiel Nitinol, umfasst oder ist. Mit Hilfe dieses die Außenwand umhüllenden Korbes kann eine maximale Expansion der Kühlkammer definiert werden.

Vorteilhafterweise umfasst das medizinische Gerät, insbesondere der Katheter, wenigstens einen Drucksensor zur Bestimmung des Blutdrucks. Eine solche Blutdruckmessung vorzugsweise während der Behandlung kann eine ständige Kontrolle des Behandlungserfolges, insbesondere bei einer Ausführung von mehreren sukzessiven Kälteablationsbehandlungen, ermöglichen, so dass bei Eintritt eines gewünschten Ergebnisses die Behandlung beendet werden kann. Das medizinische Gerät kann zusätzlich oder alternativ wenigstens eine Elektrode zur Abgabe eines Impulses für eine Anregung der renalen perivaskulären Nerven umfassen, so dass ermittelt werden kann, ob es durch eine entsprechende Stimulation zu einer Blutdruckerhöhung kommt. Wird eine solche festgestellt, insbesondere in einem nicht tolerierbaren Ausmaß, so wird die Kälteablationsbehandlung begonnen oder fortgesetzt bzw. wiederholt, bis es nach einer erneuten Stimulation zu keiner erneuten oder allenfalls zu einer tolerierbaren Blutdruckerhöhung kommt.

Eine noch weitere Ausgestaltung der Erfindung sieht vor, dass der Katheter an der Außenseite der Kühlkammer angeordnete Messelektroden für eine Widerstands- bzw. Impedanzmessung zur Erkennung des Zustands einer Denervierungsbehandlung umfasst. Auf diese Weise ist ein Wert für die Läsionstiefe ermittelbar. Überschreitet der ermittelte Wert einen vorbestimmten Grenzwert, wird die Behandlung abgebrochen, um eine übermäßige, ggf. gesundheitsgefährdende Gewebeschädigung zu vermeiden. Besonders bevorzugt werden die Elektroden zur Stimulation der renalen perivaskulären Nerven auch als Messelektroden zur Impedanzmessung verwendet, so dass keine zusätzlichen Elektroden angebracht werden müssen.

Die voranstehende Aufgabe wird weiterhin mit einem Verfahren zum Betreiben eines medizinischen Gerätes zur Denervierung gemäß dem Oberbegriff des Patentanspruchs 12 durch die kennzeichnenden Merkmale des Patentanspruchs 12 gelöst.

Das erfindungsgemäße Verfahren zum Betreiben eines medizinischen Gerätes ist zur Kryotherapie vorgesehen. Dieses medizinische Gerät, das insbesondere nach einer der vorgenannten Ausführungsformen ausgestaltet ist, umfasst einen Katheter zur Kälteablation, der einen wenigstens teilweise flexiblen, rohrförmigen Schaft zum Einführen des Katheters in ein Körpergefäß, beispielsweise in eine renale Arterie eines Patienten, und eine am distalen Ende des Schafts angeordnete und einen Endabschnitt des distalen Endes umschließende expandierbare Kühlkammer, insbesondere einen Kryoballon, aufweist. Das Kühlmedium wird in einem ersten Lumen aus einer Kühlmediumquelle vom proximalen Ende des Schafts zum distalen Ende des Schafts transportiert. Insbesondere daran anschließend wird das Kühlmedium der expandierbaren Kühlkammer mittels wenigstens einer am Ende oder einem Endbereich des ersten Lumens angeordneten Düse zugeführt. Insbesondere wiederum anschließend wird das Kühlmedium in einem zweiten Lumen vom distalen Ende des Schafts abtransportiert. Thermische Energie wird über eine Außenwand der Kühlkammer zur Durchführung einer Kryotherapie an einem Behandlungsort übertragen. Erfindungsgemäß wird das Kühlmedium infolge einer isenthalpen Druckminderung gemäß dem Joule-Thomson-Effekt beim Eintritt in das Innere der Kühlkammer expandiert und dadurch abgekühlt. Dabei kühlt das expandierte Kühlmedium im Inneren der Kühlkammer die zum gleichen Zeitpunkt in einem Endabschnitt des distalen Endes des ersten Lumens befindliche Kühlmedium durch die Oberfläche dieses Endabschnitts nach dem Gegenstromprinzip über einen Wärmeübertragungskörper ab. Weiterhin ist vorgesehen, dass das Kühlmedium auf seinem Weg vom Eintritt in das proximale Ende des ersten Lumens bis zum Inneren der Kühlkammer vorzugsweise zweimal den Aggregatszustand ändert.

Das erfindungsgemäße Verfahren ist in besonderer Weise derart ausgestaltet, dass das Kühlmedium
- im gasförmigen Aggregatszustand einem proximalen Ende des ersten Lumens zugeführt und in diesem gasförmigen Aggregatszustand zum von der Kühlkammer umschlossenen Endabschnitt des distalen Endes des ersten Lumens weitergeleitet wird,
- im Endabschnitt des distalen Endes des ersten Lumens infolge der Übertragung thermischer Energie bis wenigstens zu einem Übergang in den flüssigen Aggregatszustand abgekühlt wird,
- durch die wenigstens eine Düse aus dem ersten Lumen austritt und in das Innere der Kühlkammer eintritt, wobei beim Eintritt in das Innere der Kühlkammer eine weitere Abkühlung des Kühlmediums aufgrund des Joule-Thomson-Effekts stattfindet, und
- im Inneren der Kühlkammer sowohl zur Abkühlung des Kühlmediums im Endabschnitt des distalen Endes des ersten Lumens als auch zur Kälteablation über die Außenwand der Kühlkammer verwendet wird.

Die voranstehend aufgezeigten einzelnen Verfahrensschritte werden dabei vorzugsweise in genau dieser Reihenfolge ausgeführt.

Vorzugsweise wird das Kühlmedium innerhalb des ersten Lumens auf weniger als 15°C, vorzugsweise weniger als 10°C, bei einem im ersten Lumen aufgebauten Druck im Bereich zwischen 0,04 bis 0,06 hPa, vorzugsweise wenigstens annähernd 0,05 hPa, abgekühlt.

Es sei darauf hingewiesen, dass alle im Wege von Vorrichtungsmerkmalen beschriebenen Merkmale des medizinischen Geräts, die in diesem ausführbar sind, auch in diesem Gerät als ausgeführt beschrieben sein sollen, so dass diese vorgenannten Vorrichtungsmerkmale, sofern entsprechend geeignet, auch als Verfahrensmerkmale offenbart gelten.

Die Erfindung wird im Folgenden anhand der Zeichnungen eines Ausführungsbeispiels näher erläutert. Dabei zeigen:
- Fig. 1: eine schematische Darstellung eines Kryotherapie-Gerät mit einem Katheter;
- Fig. 2: eine vergrößerte Detailansicht gemäß Detail II in Fig. 1 vom distalen Ende des Katheters in einer Längsschnittdarstellung in einer ersten Ausführungsform;
- Fig. 3: das distale Ende gemäß Fig. 2 in seinen Einzelteilen in einer 3D-Explosionsdarstellung;
- Fig. 4a),4b): vergrößerte Detailansichten gemäß Detail II in Fig. 1 vom distalen Ende des Katheters in einer Längsschnittdarstellung gemäß einer zweiten Ausführungsform.

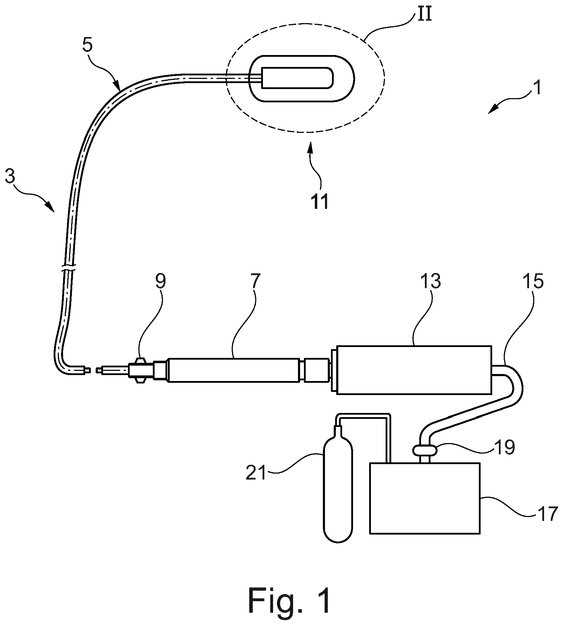

Fig. 1 zeigt eine Ausführungsform eines Kryotherapie-Gerätes 1 gemäß der vorliegenden Erfindung. Das Kryotherapie-Gerät 1 umfasst einen Katheter 3, der einen Schaft 5, einen Handhabungsgriff 7 und einen Steuerknopf 9 aufweist. Der Steuerknopf 9 ist in axialer Richtung relativ zum Handhabungsgriff 7 bewegbar, und zwar derart, dass das distale Ende 11 des Katheters 3 zur Richtungssteuerung beim Einführen des Katheters lenkbar ist.

In Fig. 1 ist eine Wärmetauschereinrichtung 13 dargestellt, die optional vorgesehen sein kann und die in diesem Fall mit dem Handhabungsgriff 7 gekoppelt ist. Mittels dieser Wärmetauschereinrichtung 13, die über einen Verbindungsschlauch 15 mit einer Steuereinheit 17 verbunden ist, ist eine Vorkühlung eines Kühlmediums vor seinem Eintritt in den Schaft 5 möglich. Ein Steuerventil 19 ist im Verbindungsschlauch angeordnet und dient zur Regelung des Gasflusses, welches als Kühlmedium dient und aus einem Gaszylinder 21 strömt. Vorzugsweise kommt als Kühlmedium Distickstoffmonoxid (N₂O) zum Einsatz, jedoch sind auch andere Gase als Kühlmedium denkbar.

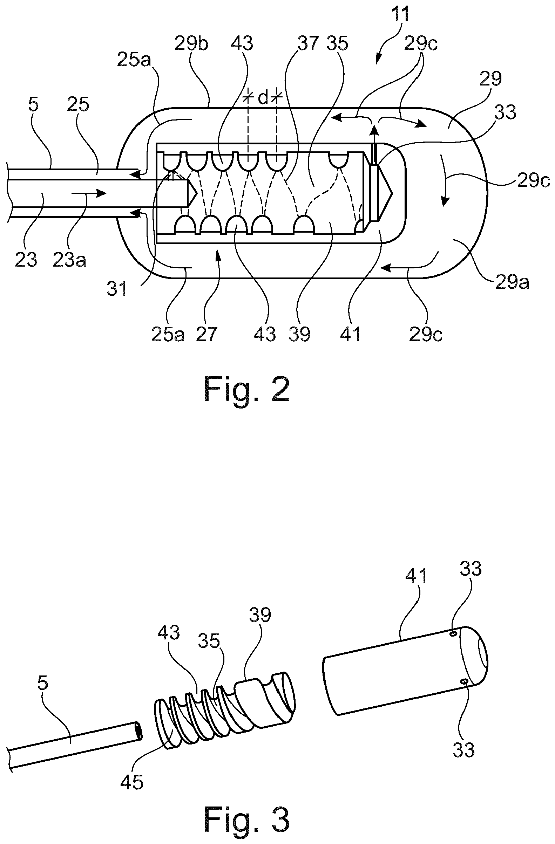

Das Steuerventil 19 dient zur Regelung des Gasflusses durch den Schaft 5 zum distalen Katheterende 11, das auch die eigentliche Behandlungseinheit zur Kälte- bzw. Kryoablation darstellt. Wie insbesondere der Längsschnittdarstellung in der vergrößerten Detailansicht in Fig. 2 zu entnehmen ist, weist der Schaft 5 ein erstes, im vorliegenden Fall ein inneres, Lumen 23 zur Zufuhr 23a bzw. zum Transport des Kühlmediums zum distalen Katheterende 11 auf. Dieses innere Lumen 23 erstreckt sich von der Kühlmediumquelle 21, oder im vorliegenden Beispiel von der Wärmetauschereinrichtung 13, bis zum distalen Katheterende 11. Vom distalen Katheterende 11 wird das durch die Behandlung, welche weiter unten genauer beschrieben wird, "verbrauchte" Kühlmedium durch ein ebenfalls im Schaft 5 angeordnetes zweites, im vorliegenden Fall ein äußeres, Lumen 25 zur Ausleitung 25a gebracht, wobei die Ausleitung 25a üblicherweise in die Narkosegasabsaugung des Behandlungsraums stattfindet. Alternativ kann das Kühlmedium auch im Wege eines Kreislaufes wieder zurückgeführt werden, so dass anstelle des Gaszylinders 21 ein Gasreservoir mit Rückspeisung von Kühlmedium aus dem äußeren Lumen 25 grundsätzlich denkbar ist.

Die vergrößerte Längsschnittdarstellung gemäß Fig. 2 illustriert nähere Details des distalen Katheterendes 11, welches im Allgemeinen einen Wärmeübertragungskörper 27 und einen diesen Wärmeübertragungskörper umhüllenden expandierbaren Kryoballon 29 umfasst. Im Wesentlichen wird durch den Wärmeübertragungskörper 27 das Kühlmedium von einer an einer ersten Seite des Wärmeübertragungskörpers 27 gelegenen Eingangsseite zu einer an einer der ersten Seite gegenüberliegenden zweiten Seite des Wärmeübertragungskörpers 27 angeordneten Ausgangsseite hindurchgeleitet. Zu diesem Zweck ist an der Eingangsseite ein Kanaleingang 31 angeordnet, in den der Teil des im Schaft 5 geführten inneren Lumens 23 mündet. Nach Durchströmen des Wärmeübertragungskörpers 27 tritt das Kühlmedium durch eine Düse 33 ins Innere 29a des von einer Ballonhülle 29b begrenzten Kryoballons 29 ein. Durch den Eintritt des gasförmigen Kühlmediums ins Innere 29a des Kryoballons 29 wird dieser expandiert und die Ballonhülle 29b dehnt sich entsprechend. Im Inneren 29a des Kryoballons 29 verteilt sich das Kühlmedium wie bei 29c angedeutet und strömt zur ersten Seite des Wärmeübertragungskörpers 27 zurück, die nächstgelegen zum Schaft 5 ist, so dass das Kühlmedium wie bei 25a angedeutet durch das äußere Lumen 25 ausgetragen wird. Auf dem Weg durch das Innere des Kryoballons 29 kühlt das Kühlmedium den Kryoballon 29 ab, so dass eine entsprechende Temperatur auch an der wegen ihrer geringen Dicke besonders zur Wärmeübertragung geeigneten Ballonhülle 29b vorliegt. Im Behandlungsfall berührt dann die Ballonhülle 29b beispielsweise die zu abladierenden renalen perivaskulären Nerven, deren Position mit Katheter 3 über die jeweilige Nierenarterie angesteuert wurde.

In der Längsschnittdarstellung von Fig. 2 ist auch erkennbar, welchen Weg das Kühlmedium durch den Wärmeübertragungskörper 27 nimmt. Nach Eintritt in den Wärmeübertragungskörper 27 am Kanaleingang 31 durchläuft das innere Lumen 23 nahe einer Grenzfläche zum Inneren 29a des Kryoballons 29 einen schraubenförmigen Weg, der um eine Kernstruktur 35 geführt ist, wie in Fig. 2 bei 37 als Hilfslinien angedeutet.

Anhand der 3D-Explosionsdarstellung gemäß Fig. 3 ist die Herstellung des zylinderförmig ausgebildeten Wärmeübertragungskörpers 27 erkennbar. Dieser besteht im Wesentlichen aus zwei Elementen, einem inneren Element 39 und einem äußeren Element 41. Im ebenfalls zylinderförmig ausgestalteten inneren Element 39 ist an dessen Mantelfläche das innere Lumen 23 als Helixstruktur insbesondere während eines Produktionsschritts im Wege eines Fräsvorgangs oder mit Hilfe eines Laserstrahls eingebracht, wodurch der Weg, den das Kühlmedium durch den Wärmeübertragungskörper 27 nimmt, definiert ist. Gemäß einer besonders kostengünstigen Variante kann das Kernelement auch im Spritzgussverfahren hergestellt sein. Wie in Fig. 2 und 3 dargestellt, ist die eingebrachte Helixstruktur als schraubenförmige Nut 43 mit U-förmigem Querschnitt dargestellt. Die Ganghöhe der Helixstruktur, also der Abstand d zwischen den Zentren einer gemäß Fig. 2 ersten Schnittfläche und einer zweiten Schnittfläche des inneren Lumens 23, wobei zwischen erster und zweiter Schnittfläche ein einfacher vollständiger Umlauf des ersten Lumens 23 um die Kernstruktur 35 liegt, ist derart eingestellt, dass sich eine besonders große Grenz- bzw. Oberfläche zwischen innerem Lumen 23 und dem Inneren 29a des Kryoballons 29 zum Zwecke eines besonders großen Wärmeübergangs einstellt.

Nach dem Einbringen der Helixstruktur an der Mantelfläche des inneren Elements 39 wird der Wärmeübertragungskörper 27 fertiggestellt, indem das ebenfalls zylindrische, hutförmige äußere Element 41 über das innere Element 39 gesteckt, insbesondere aufgepresst, wird. Das äußere Element 41 umschließt das innere Element 39 passgenau, d. h. ohne Spiel zwischen diesen beiden Elementen 39, 41. Auf diese Weise ist auch das helixförmige innere Lumen 23 im Wärmeübertragungskörper 27 definiert.

In Fig. 3 ist bei 45 noch die Eintrittsöffnung in das helixförmige innere Lumen 23 nahe beim Kanaleingang 31 angedeutet. Es erfolgt somit nach dem Kanaleingang 31 eine Umlenkung des inneren Lumens 23 um etwa 90°, so dass der Eintritt des Kühlmediums in die Helixstruktur in radialer Richtung erfolgt. Außerdem sind bei der Ausgestaltung gemäß Fig. 3 mehrere Düsen 33 vorgesehen, die zu einem gleichmäßigeren Eintrag von Kühlmedium in das Innere 29a des Kryoballons 29 und auch zu einer weiteren Kühleffizienzsteigerung beitragen, da an jeder einzelnen Düse 33 eine entsprechende Kältemenge erzeugt wird.

Aus der Darstellung gemäß Fig. 2 wird auch die Wirkweise des Kryoballons 29 und des von diesem umschlossenen Wärmeübertragungskörper 27 deutlich. Das Kühlmedium wird im gasförmigen Zustand ausgehend vom Gaszylinder 21 als Kühlmediumquelle über das innere Lumen 23 in den Wärmeübertragungskörper 27 eingetragen und durchläuft das darin angeordnete helixförmige innere Lumen 23 bis zum Austritt in das Innere 29a des Kryoballons 29. Aufgrund des Joule-Thomson-Effekts wird das Kühlmedium beim Austritt aus der Düse 33 entspannt und dadurch abgekühlt, so dass die Temperatur des Kühlmediumanteils im Inneren 29a des Kryoballons 29, also in der Nut 43, niedriger ist als die Temperatur des Kühlmediums im helixförmigen ersten Lumen 23 zum gleichen Zeitpunkt. Aufgrund dieser Temperaturdifferenz zwischen dem Inneren 29a des Kryoballons 29 und dem inneren Lumen 23 findet ein Austausch von Wärmeenergie an deren Grenz- bzw. Oberfläche statt, so dass sich die Temperatur des Kühlmediums im inneren Lumen 23 weiter reduziert. Dadurch, dass das Kühlmedium aus dem Inneren 29a des Kryoballons 29 kontinuierlich über das äußere Lumen 25 ausgetragen wird, wobei neue, in der Temperatur weiter verringerte Kühlmedium aus dem inneren Lumen 23 in der Helixstruktur nachgeführt werden, erfolgt sukzessive eine weitere Temperaturverringerung im Inneren 29a des Kryoballons 29. Durch den weiteren Wärmeaustausch über die Grenz- bzw. Oberfläche zwischen dem Inneren 29a des Kryoballons 29 und dem inneren Lumen 23 im Wärmeübertragungskörper 27 wird ein Temperaturwert erreicht, bei dem sich das Kühlmedium im inneren Lumen 23 im Wärmeübertragungskörper 27 verflüssigt. Wenn nun dieses verflüssigte Kühlmedium über die Düse 33 in das Innere 29a des Kryoballons 29 eingesprüht wird, so nimmt es durch die dadurch bewirkte Entspannung wieder den gasförmigen Zustand an, wodurch eine Abkühlung des Kühlmediums stattfindet, die sowohl auf den Joule-Thomson-Effekt beruht, als auch auf den Phasenübergang von flüssig nach gasförmig, durch den Wärmeenergie aus der Umgebung der Düse 33 entnommen wird, was also zu einer weiteren Abkühlung des Kühlmediums führt. Auf diese Weise ist der Kühleffekt im beschriebenen System weiter gesteigert, so dass für die Kryoablationsbehandlung eine ausreichende Kältemenge zur Verfügung steht. Wenn sich der Querschnitt der Nut entlang des Strömungsweges innerhalb des ersten Lumensabschnittes und insbesondere im Wärmeübertragungskörper ändert, kann der Übergang von flüssig auf gasförmig auch schon vor dem Austritt aus der Düse in die Kühlkammer erfolgt sein.

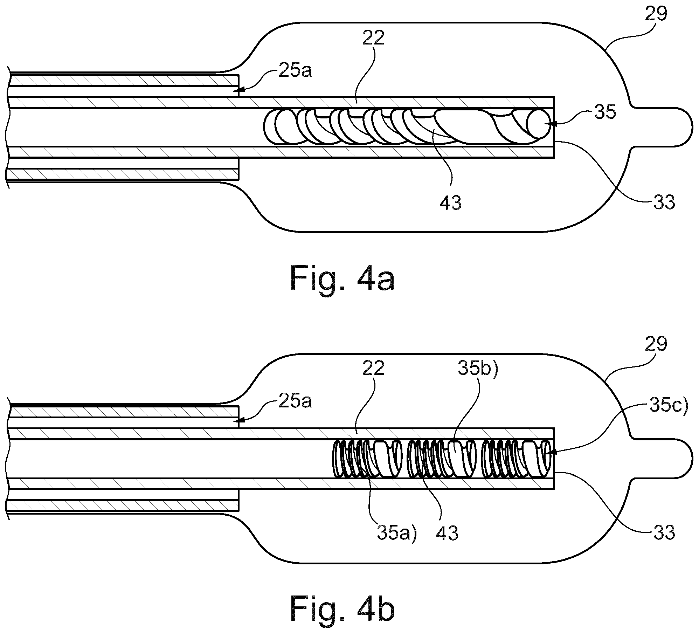

Anhand der Darstellung gemäß Fig. 4a) ist die Herstellung des zylinderförmig ausgebildeten Wärmeübertragungskörpers 27 gemäß einer zweiten Ausführungsform erkennbar.

Auch hier wird der Wärmeübertragungskörper im Wesentlichen aus zwei Elementen, einem inneren Element und einem äußeren Element gebildet.

Das innere Element ist dabei als Kern 35 ausgebildet, welcher eine helixförmig umlaufende Nut 43 besitzt. Dieser Kern wird bei der Herstellung des Gerätes passgenau in das innere Lumen 23 des Katheters geschoben und ggf. verklebt. Das innere Lumen wird durch die Wandung des schlauchförmigen Elementes 22 gebildet. Dabei bildet die Wandung des schlauchförmigen Elementes 22 zusammen mit dem Kern 35 den Wärmeübertragungskörper 27.

Die Struktur des Kerns kann dabei der in Fig. 3 dargestellten Struktur des Kerns entsprechen. Der erste Lumenabschnitt des ersten Lumens wird in dieser Ausführung durch die Nut 43 des Kerns 35 und die den Kern 35 umschließende mantelförmige Wandung des schlauchförmigen Elementes 22 gebildet.

Auch hier kann vorgesehen sein, dass sich die Tiefe der Nut ändert. So kann vorgesehen sein, dass sich der Querschnitt der Nut von einem Punkt des minimalen Querschnittes Richtung Düsenaustritt erweitert. Im Bereich der Stelle mit minimalem Querschnitt können gemäß einer weiteren Ausführung auch eine oder mehrere Aussparungen in der Wandung des schlauchförmigen Elementes vorgesehen sein, sodass Kühlmedium aus der Kühlkammer durch den entsprechend des Venturi-Effektes entstandenen Unterdruck angesaugt und am Ende der Düse in die Kühlkammer ausgestoßen werden könnten.

Figur 4b)zeigt eine Abwandlung der in Figur 4a) dargestellten Ausführungsform. Auch hier wird der Wärmeübertragungskörper 27 durch die Wandung des schlauchförmigen Elementes 22 zusammen mit dem Kern 35 gebildet. Im Unterschied zu Figur 4a) ist der Kern aus einer Mehrzahl von Segmenten gebildet. Hierdurch wird der Katheter insgesamt weniger steif und kann auf diese Weise besser den Behandlungsort erreichen.

Schließlich sei noch darauf hingewiesen, dass mit Fig. 1 bis 4 die Einzelheiten des Kryotherapie-Geräts 1 in einigen Teilen lediglich schematisch dargestellt sind. Insbesondere in der Darstellung gemäß Fig. 2 sind nur die für das Verständnis der Erfindung notwendigen Merkmale und Komponenten illustriert. Für den praktischen Einsatz des Katheters sind weitere Elemente erforderlich oder hilfreich. Insbesondere ein Vorsehen eines J-wires zur Handhabung des Katheters 3 beim Einführen in den Körper des Patienten, also die Einführung z.B. in die renale Arterie, wie beispielsweise in Fig. 2 der EP 3 708 100 A1 mit Bezugszeichen 7 gezeigt, ist für die praktische Ausgestaltung des Katheters hilfreich. Weiterhin sind in den Darstellungen auch für eine erfolgreiche Behandlung nützliche Elemente bzw. Komponenten wie die allgemein erwähnten Drucksensoren und Elektroden zur Stimulierung von Nervenzellen oder Messelektroden nicht dargestellt, sollen aber im Rahmen der Erfindung auch bei den illustrierten Ausbildungsformen ebenfalls als offenbart gelten.

### Bezugszeichenliste

- 1: Kryotherapie-Gerät
- 3: Katheter
- 5: Schaft
- 7: Handhabungsgriff
- 9: Steuerknopf
- 11: distales Ende
- 13: Wärmetauschereinrichtung
- 15: Verbindungsschlauch
- 17: Steuereinheit
- 19: Steuerventil
- 21: Gaszylinder
- 22: schlauchförmiges Element
- 23: inneres Lumen
- 23a: Kühlmediumzufuhr
- 25: äußeres Lumen
- 25a: Kühlmediumausleitung
- 27: Wärmeübertragungskörper
- 29: Kryoballon
- 29a: Balloninneres
- 29b: Ballonhülle
- 29c: Strömung im Balloninneren
- 31: Kanaleingang
- 33: Düse
- 35: Kernstruktur
- 37: Schraubenlinie
- 39: inneres Element
- 41: äußeres Element
- 43: Nut
- 45: Eintrittsöffnung
- d: Streckenabschnitt

## Patentansprüche

1. Medizinisches Gerät (1) zur Kryotherapie, umfassend einen Katheter (3), wobei der Katheter (3) folgendes aufweist:
1.1 einen wenigstens teilweise flexiblen, rohrförmigen Schaft (5) zum Einführen des Katheters (3) in ein Körpergefäß, wobei der Schaft (5) ein erstes, vorzugsweise ein inneres, Lumen (23) zum Transportieren eines Kühlmediums zu einem distalen Ende (11) des Schafts (5) und ein zweites, vorzugsweise ein äußeres und insbesondere das innere Lumen (23) umgebendes, Lumen (25) zum Abtransportieren des Kühlmediums vom distalen Ende (11) des Schafts (5) umfasst, wobei das erste Lumen (23) an einem proximalen Ende des Schafts (5) mit einer Kühlmediumquelle (21) verbunden ist,
1.2 eine expandierbare Kühlkammer (29), insbesondere einen Kryoballon,
- welche am distalen Ende (11) des Schafts (5) angeordnet ist und einen Endabschnitt des distalen Endes (11) des Schafts (5) und/oder einen einem Endabschnitt des distalen Endes (11) des Schafts (5) zugeordneten ersten Lumenabschnitt des ersten Lumens (23) umschließt,
- welcher das Kühlmedium über das erste Lumen (23) mittels wenigstens einer Düse (33) zuführbar ist, und
- aus welcher das Kühlmedium über das zweite Lumen (25) abtransportierbar ist,
- wobei die Kühlkammer (29), insbesondere unter Zufuhr des Kühlmediums, expandierbar ist und wobei thermische Energie über eine Außenwand (29b) der Kühlkammer (29) übertragbar ist, sodass eine Kryotherapie an einem Behandlungsort durchführbar ist, **dadurch gekennzeichnet, dass**
- der Endabschnitt am distalen Ende (11) des Schafts (5) einen Wärmeübertragungskörper (27) umfasst oder einen Wärmeübertragungskörper (27) bildet, welcher ein inneres Element (39) und ein das innere Element (39), insbesondere hut-, kappen- oder mantelförmig, umschließendes äußeres Element (41) aufweist, wobei durch das Zusammenwirken des inneren Elements (39) und des äußeren Elements (41) der erste Lumenabschnitt des ersten Lumens (23) gebildet ist und
- die wenigstens eine Düse (33) eine den Joule-Thomson-Effekt nutzende Düse ist und wenigstens ein Teil des Endabschnitts am distalen Ende (11) des Schafts (5), insbesondere wenigstens ein überwiegender Teil des von der expandierbaren Kühlkammer (29) umschlossenen ersten Lumenabschnitts des ersten Lumens (23), mit dem Inneren (29a) der Kühlkammer (29) in wärmeleitendem Kontakt zur Kühlung des im ersten Lumen (23) geführten Kühlmediums durch das dem Inneren (29a) der Kühlkammer (29) zugeführte Kühlmedium ist.

2. Medizinisches Gerät (1) nach Anspruch 1,
**dadurch gekennzeichnet, dass**
das von der Kühlmediumquelle (21) dem ersten Lumen (23) zugeführte Kühlmedium gasförmig ist und dass durch die Kühlung des Kühlmediums im ersten Lumen (23), insbesondere im ersten/distalen Lumenabschnitt des ersten Lumens (23), infolge des Übergangs von thermischer Energie zwischen dem Inneren (29a) der Kühlkammer (29) und dem ersten Lumen (23), insbesondere dem ersten Lumenabschnitt des ersten Lumens (23), das insbesondere bis zum Eintreten in den Kühlkammerbereich gasförmige Kühlmedium in den flüssigen Aggregatszustand überführbar ist.

3. Medizinisches Gerät (1) nach Anspruch 1 oder 2,
**dadurch gekennzeichnet, dass**
der erste Lumenabschnitt des ersten Lumens (23) helixförmig um eine Kernstruktur (35) gewickelt ist, wobei die Kernstruktur (35) und die helixförmige Anordnung des ersten Lumenabschnitts insbesondere von einem Wärmeübertragungskörper (27) umfasst oder eingeschlossen sind bzw. ein Teil davon sind.

4. Medizinisches Gerät (1) nach Anspruch 3,
**dadurch gekennzeichnet, dass**
die Helixform derart ausgestaltet ist, dass das erste Lumen (23), insbesondere das Kühlmedium im ersten Lumen (23), im ersten Lumenabschnitt nach einem einfachen, vollständigen Umlauf um die Kernstruktur (35) einen Streckenabschnitt (d) in axialer Richtung durchläuft, der nicht größer als der zweifache Durchmesser, vorzugsweise geringfügig größer als der einfache Durchmesser, des ersten Lumens (23) ist.

5. Medizinisches Gerät (1) nach einem der Ansprüche 1 bis 4 ,
**dadurch gekennzeichnet, dass**
der erste Lumenabschnitt des ersten Lumens (23) als Vertiefung an der, insbesondere als Zylindermantelfläche ausgebildeten, Oberfläche des inneren Elements (39) gebildet ist, wobei die Vertiefung vorzugsweise als helixförmige oder spiralförmige Nut (43) und/oder mit U-förmigem Querschnitt ausgebildet ist.

6. Medizinisches Gerät (1) nach einem der Ansprüche 1 bis 5,
**dadurch gekennzeichnet, dass** der erste Lumenabschnitt des ersten Lumens (23) durch eine in das erste Lumen (23) am distalen Ende eingeschobene Kernstruktur mit helixförmiger Vertiefung zusammen mit dem das erste Lumen (23) umfassenden schlauchförmigen Element (22)gebildet ist.

7. Medizinisches Gerät (1) nach einem der Ansprüche 1 bis 6,
**dadurch gekennzeichnet, dass**
in der Kühlkammer (29), insbesondere im Kryoballon, durch den Austritt des Kühlmediums aus der Düse (33) ein gegenüber dem Fluiddruck im ersten Lumen (23) niedrigerer Druck einstellbar ist, welcher vorzugsweise kleiner als ein der Festigkeit der Außenwand (29b) der Kühlkammer (29) zugeordneter oder zuordenbarer Maximaldruck ist, wobei die Düse (33) insbesondere eine Venturidüse ist.

8. Medizinisches Gerät (1) nach einem der Ansprüche 1 bis 7,
**dadurch gekennzeichnet, dass**
die Außenwand (29b) der Kühlkammer (29), insbesondere die Hülle des Kryoballons, aus einem dünnwandigen dehnbaren Material gebildet und doppelwandig ausgestaltet ist, wobei insbesondere wenigstens ein Sensor zur Erfassung des Zustands der Außenwand (29b) umfasst ist, beispielsweise zur Überwachung eines Parameters im durch die innere und die äußere Wand definierten Zwischenraum der Außenwand (29b).

9. Medizinisches Gerät (1) nach einem der Ansprüche 1 bis 8,
**dadurch gekennzeichnet, dass**
die Außenwand (29b) der Kühlkammer (29), insbesondere die Hülle des Kryoballons, wenigstens im Betriebszustand des medizinischen Gerätes (1)
aus einem Material umfassend eine Formgedächtnislegierung gebildet ist, und/oder
von einem, insbesondere aus einem Material umfassend eine Formgedächtnislegierung gebildeten, Korb wenigstens teilweise umgeben ist.

10. Medizinisches Gerät (1) nach einem der Ansprüche 1 bis 9,
**dadurch gekennzeichnet, dass**
das medizinische Gerät (1), insbesondere der Katheter (3), wenigstens einen Drucksensor zur Bestimmung des Blutdrucks, insbesondere während der Behandlung, und/oder wenigstens eine Elektrode zur Abgabe eines elektrischen Impulses für eine Anregung von Nerven umfasst.

11. Medizinisches Gerät (1) nach einem der Ansprüche 1 bis 10,
**dadurch gekennzeichnet, dass**
das medizinische Gerät (1), insbesondere der Katheter (3), vorzugsweise an der Außenseite der Kühlkammer (29) angeordnete Messelektroden für eine Widerstandsmessung zur Erkennung des Zustands einer Denervierungsbehandlung umfasst.

12. Verfahren zum Betreiben eines medizinisches Gerätes (1) zur Denervierung, insbesondere von renalen perivaskulären Nerven, wobei das Gerät (1) einen Katheter (3) zur Kälteablation umfasst, der einen wenigstens teilweise flexiblen, rohrförmigen Schaft (5) zum Einführen des Katheters (3) in ein Körpergefäß, insbesondere in eine renale Arterie eines Patienten, und eine am distalen Ende (11) des Schafts (5) angeordnete und einen Endabschnitt des distalen Endes (11) umschließende expandierbare Kühlkammer (29), insbesondere einen Kryoballon, aufweist, wobei das Kühlmedium
- in einem ersten Lumen (23) aus einer Kühlmediumquelle (21) vom proximalen Ende des Schafts (5) zum distalen Ende (11) des Schafts (5) transportiert wird,
- der expandierbaren Kühlkammer (29) mittels wenigstens einer am Ende oder einem Endbereich des ersten Lumens (23) angeordneten Düse (33) zugeführt wird, und
- in einem zweiten Lumen (25) vom distalen Ende (11) des Schafts (5) abtransportiert wird,
- wobei thermische Energie über eine Außenwand (29b) der Kühlkammer (29) zur Durchführung einer Kryotherapie an einem Behandlungsort übertragen wird, und
- wobei das medizinische Gerät (1) insbesondere nach einem der Ansprüche 1 bis 11 ausgebildet ist,
**dadurch gekennzeichnet, dass**
- das Kühlmedium infolge einer isenthalpen Druckminderung gemäß dem Joule-Thomson-Effekt beim Eintritt in das Innere (29a) der Kühlkammer (29) expandiert und dadurch abgekühlt wird, wobei das expandierte Kühlmedium im Inneren (29a) der Kühlkammer (29) das zum gleichen Zeitpunkt in einem Endabschnitt des distalen Endes (11) des ersten Lumens (23) befindliche Kühlmedium durch die Oberfläche dieses Endabschnitts nach dem Gegenstromprinzip abkühlt, und wobei das Kühlmedium auf seinem Weg vom Eintritt in das proximale Ende des ersten Lumens (23) bis zum Inneren (29a) der Kühlkammer (29) vorzugsweise zweimal den Aggregatszustand ändert.

13. Verfahren nach Anspruch 12,
**dadurch gekennzeichnet, dass**
das Kühlmedium
- im gasförmigen Aggregatszustand einem proximalen Ende des ersten Lumens (23) zugeführt und in diesem gasförmigen Aggregatszustand zum von der Kühlkammer (29) umschlossenen Endabschnitt des distalen Endes (11) des ersten Lumens (5) weitergeleitet wird,
- im Endabschnitt des distalen Endes (11) des ersten Lumens (5) infolge thermischer Energieübertragung bis wenigstens zu einem Übergang in den flüssigen Aggregatszustand abgekühlt wird,
- durch die wenigstens eine Düse (33) aus dem ersten Lumen (5) austritt und in das Innere (29a) der Kühlkammer (29) eintritt, wobei beim Eintritt in das Innere (29a) der Kühlkammer (29) eine weitere Abkühlung des Kühlmediums aufgrund des Joule-Thomson-Effekts stattfindet, und
- im Inneren (29a) der Kühlkammer (29) sowohl zur Abkühlung des Kühlmediums im Endabschnitt des distalen Endes (11) des ersten Lumens (5) als auch zur Kälteablation über die Außenwand (29b) der Kühlkammer (29) verwendet wird.

14. Verfahren nach Anspruch 12 oder 13,
**dadurch gekennzeichnet, dass**
das Kühlmedium innerhalb des ersten Lumens (23) auf weniger als 15°C, vorzugsweise weniger als 10°C, bei einem im ersten Lumen (23) aufgebauten Druck im Bereich zwischen 0,04 bis 0,06 hPa, vorzugsweise wenigstens annähernd 0,05 hPa, abgekühlt wird.
